# EUROPÄISCHE PATENTANMELDUNG

(11) **EP 4 497 742 A1**
(43) Veröffentlichungstag der Anmeldung: **29.01.2025**
(21) Anmeldenummer: 23020353.1
(22) Anmeldetag: 25.07.2023
(51) Int. Cl.: C07C 5/48, C07C 7/00, C07C 7/04, C07C 11/04, F25J 3/00

(54) **VERFAHREN UND ANLAGE ZUR HERSTELLUNG EINES ODER MEHRERER SYNTHESEPRODUKTE**

(71) Anmelder: Linde GmbH, 82049 Pullach (DE)
(72) Erfinder: Fritz, Helmut, 82049 Pullach (DE); Meiswinkel, Andreas, 82049 Pullach (DE); Kracker, Gunther, 82049 Pullach (DE); Tota, Desislava, 82049 Pullach (DE); Bermejo, Ricardo, 82049 Pullach (DE); Schubert, Martin, 82049 Pullach (DE)
(74) Vertreter: Reuß, Stephanie

(57) **Zusammenfassung**

Es wird ein Verfahren (100) zur Herstellung eines oder mehrerer Syntheseprodukte vorgeschlagen, bei dem Ethan als eine erste Einsatzverbindung in einem ersten Einsatzstrom (11) unter Erhalt eines Ethylen als ein erstes Produktolefin enthaltenden ersten Produktstroms (12) einer oxidativen Dehydrierung (110) unterworfen wird, und eine Sauerstoff und Kohlenstoff enthaltende zweite Einsatzverbindung in einem zweiten Einsatzstrom (21) unter Erhalt eines das erste und/oder ein zweites Produktolefin enthaltenden zweiten Produktstroms (22) einem oder mehreren katalytischen Umsetzungsschritten (210) unterworfen wird. Hierbei ist vorgesehen, dass zumindest ein Teil des ersten Produktstroms (12) unter Erhalt eines ersten Folgestroms (13, 14) einem oder mehreren ersten Reinigungs- und/oder Trennschritten (310) unterworfen wird, zumindest ein Teil des zweiten Produktstroms (21) unter Erhalt eines zweiten Folgestroms (22, 23) keinem, einem oder mehreren zweiten Reinigungs- und/oder Trennschritten (310) unterworfen wird, und zumindest ein Teil des ersten Folgestroms (13, 14) und zumindest ein Teil des zweiten Folgestroms zur Bildung eines dritten Einsatzstroms verwendet wird, wobei der dritte Einsatzstrom einem oder mehreren dritten Reinigungs- und/oder Trennschritten (310) zugeführt wird. Eine entsprechende Anlage ist ebenfalls Gegenstand der Erfindung.

## Beschreibung

Die Erfindung betrifft ein Verfahren und eine Anlage zur Herstellung eines oder mehrerer Syntheseprodukte.

### Hintergrund

Herkömmlicherweise wird ein überwiegender Anteil des industriell hergestellten Ethylens und Propylens durch Steamcracking (Dampfspalten) in entsprechenden Steamcrackern gewonnen. Steamcracking ist jedoch ausgesprochen energieintensiv und beruht herkömmlicherweise auf dem Einsatz fossiler Rohstoffe, wie z.B. Ethan, Propan, Flüssiggas (engl. Liquefied Petroleum Gas, LPG), Naphtha oder höheren Kohlenwasserstofffraktionen. Zudem entsteht beim Steamcracken in Abhängigkeit vom Einsatzmaterial typischerweise ein gewisser Anteil an ggf. unerwünschten weiteren Komponenten, wie z.B. Aromaten.

Vor diesem Hintergrund gewinnen alternative Verfahren zur Olefinerzeugung zunehmend an Bedeutung. Hierzu gehören selektive Verfahren, wie z.B. die oxidative Dehydrierung (engl. Oxidative Dehydrogenation, ODH), die im Fall von Ethan als Einsatzmaterial (engl. Oxidative Dehydrogenation of Ethane, ODHE) neben einer gewissen Koppelproduktion von ebenfalls wertvoller Essigsäure sehr selektiv Ethylen erzeugt. Auch Olefinerzeugungsverfahren, die von anderen Einsatzstoffen ausgehen, sind bekannt. Es handelt sich beispielsweise um die Herstellung von Fischer-Tropsch-Produkten, insbesondere in der Umsetzung zu Olefinen (engl. Fischer-Tropsch to Olefines, FTTO oder FTO), die von Synthesegas ausgeht, die Umsetzung von Methanol oder Dimethylether zu Olefinen bzw. Propylen (engl. Methanol to Olefins, MTO, bzw. Methanol to Propylene, MTP), die mit den entsprechenden Verbindungen gespeist wird, oder auch die hydrierende Umsetzung von kohlendioxidhaltigen Einsatzströmen. Methanol und Dimethylether können dabei ihrerseits ebenfalls ausgehend von Synthesegas oder kohlendioxidhaltigen Einsatzströmen erzeugt werden.

Allerdings erzeugen die genannten alternativen Olefinerzeugungsverfahren (außer der ODHE) eine breite Produktverteilung, wobei oftmals der Schwerpunkt auf der Erzeugung von Olefinen mit drei Kohlenstoffatomen liegt und keine direkte Verwertung für Verbindungen mit zwei Kohlenstoffatomen (Ethylen und insbesondere Ethan) möglich ist. Dies kann darauf zurückzuführen sein, dass für einen großtechnischen Folgeprozess zu geringe Mengen entsprechender Verbindungen mit zwei Kohlenstoffatomen gebildet werden. Solche Folgeprozesse können beispielsweise die Herstellung von Polyethylen aus Ethylen, die Herstellung von Vinylacetatmonomer durch die selektive Gasphasenoxidation von Essigsäure mit Ethylen, oder die Herstellung von Ethylenoxid oder Ethylenglycol aus Ethylen umfassen.

Prinzipiell können solche "Nebenströme" zwar in geeigneten petrochemischen Komplexen verwertet werden, wie eben einem Steamcracker. Nach heutigem Stand der Technik werden Steamcracker jedoch vorteilhafterweise in sehr großen Maßstäben erstellt (typischerweise mit mehr als 1.000 Kilojahrestonnen Ethylenkapazität). Bei einer typischen Anlage zur Umsetzung von Methanol zu Olefinen fällt eine Ethanfraktion als zunächst zwar prinzipiell gut geeigneter Steamcrackereinsatz an, allerdings eher in einer deutlich kleineren Größenordnung von lediglich ca. 1 bis 50 Kilojahrestonnen. Vor diesem Hintergrund ist also allenfalls eine Integration mit bestehender Infrastruktur oder eben an sehr großen Standorten sinnvoll möglich.

Abgesehen von einer Pipelineinfrastruktur ist zudem der Transport der benötigten Kohlenwasserstoffe mit zwei Kohlenstoffatomen, insbesondere von niedrig bewertetem Ethan, typischerweise nicht praktikabel. Demgegenüber lassen sich schwerere Nebenprodukte der Olefinerzeugungsverfahren (Propan, Kohlenwasserstoffe mit vier Kohlenstoffatomen) z.B. relativ leicht verflüssigen und z.B. als LPG transportieren. Hierfür steht eine etablierte Logistik und Infrastruktur zur Verfügung.

Es existiert also derzeit keine geeignete Lösung für eine stoffliche Verwertung insbesondere der Fraktion von Kohlenwasserstoffen mit zwei Kohlenstoffatomen aus solchen alternativen Olefinerzeugungsverfahren außerhalb von sehr großen petrochemischen Komplexen bzw. außerhalb von petrochemischen Komplexen mit sehr spezifischen Bedingungen.

Es besteht daher weiterhin der Bedarf nach Lösungen, die in entsprechenden Szenarien Verbesserungen schaffen.

### Kurzfassung

Vor diesem Hintergrund werden ein Verfahren und eine Anlage zur Herstellung eines oder mehrerer Syntheseprodukte mit den jeweiligen Merkmalen der unabhängigen Patentansprüche vorgeschlagen. Ausgestaltungen sind Gegenstand der abhängigen Patentansprüche und der nachfolgenden Beschreibung.

Das vorgeschlagene Verfahren zur Herstellung eines oder mehrerer Syntheseprodukte umfasst, dass Ethan als eine erste Einsatzverbindung in einem ersten Einsatzstrom unter Erhalt eines Ethylen als ein erstes Produktolefin enthaltenden ersten Produktstroms einer oxidativen Dehydrierung, d.h. einer ODH bzw. ODHE, unterworfen wird, und dass eine Sauerstoff und Kohlenstoff enthaltende zweite Einsatzverbindung in einem zweiten Einsatzstrom unter Erhalt eines das erste und/oder ein zweites Produktolefin enthaltenden zweiten Produktstroms einem oder mehreren katalytischen Umsetzungsschritten unterworfen wird. Hierbei ist vorgesehen, dass zumindest ein Teil des ersten Produktstroms unter Erhalt eines ersten Folgestroms einem oder mehreren ersten Reinigungs- und/oder Trennschritten unterworfen wird, zumindest ein Teil des zweiten Produktstroms unter Erhalt eines zweiten Folgestroms keinem, einem oder mehreren zweiten Reinigungs- und/oder Trennschritten unterworfen wird (der oder die zweiten Reinigungs- und/oder Trennschritte sind also optional), und zumindest ein Teil des ersten Folgestroms und zumindest ein Teil des zweiten Folgestroms zur Bildung eines dritten Einsatzstroms verwendet werden, wobei der dritte Einsatzstrom einem oder mehreren dritten Reinigungs- und/oder Trennschritten zugeführt wird.

Nachfolgend wird anstelle von Verfahrensschritten auch von entsprechenden Vorrichtungskomponenten gesprochen, um die vorliegende Erfindung und Ausgestaltungen hiervon zu beschreiben. Insbesondere betrifft dies die "Reinigungsund Trennschritte", die in entsprechenden "Reinigungs- und Zerlegungsteilen", also Anlagenteilen von entsprechenden Vorrichtungen gemäß Ausgestaltungen der Erfindung durchgeführt werden. Die entsprechenden Erläuterungen betreffen Verfahrensschritte und Vorrichtungskomponenten in gleicher Weise.

Die Erfindung und ihre Ausgestaltungen lösen, wie auch unten im Detail erläutert, mehrere Probleme bekannter Verfahren durch die Verknüpfung des einen oder der mehreren, die Sauerstoff und Kohlenstoff enthaltende zweite Einsatzverbindung umsetzenden katalytischen Umsetzungsschritte mit einer ODHE, wobei überraschenderweise signifikante Vorteile in der Gestaltung des Zerlegungsteils erzielt werden können. Die Erfindung und ihre Ausgestaltungen ermöglichen eine vorteilhafte Nutzung von leichten Kohlenwasserstoffen, insbesondere von Ethan, aus dem einen oder den mehreren katalytischen Umsetzungsschritten durch eine vorteilhafte Kombination mit einer ODHE, wobei in Ausgestaltungen der Erfindung insbesondere auch zwischen dem ersten und zweiten Produktstrom abweichende Kohlendioxid-, Sauerstoff- und Acetylengehalte berücksichtigt werden können.

Nachfolgend wird anstelle von "einem oder mehreren katalytischen Umsetzungsschritten" vereinfachend auch von einem "Olefinerzeugungsverfahren" oder "alternativen Olefinerzeugungsverfahren" gesprochen, wobei diese Begriffe synonym verwendet werden. Das (alternative) Olefinerzeugungsverfahren unterscheidet sich durch die katalytische Umsetzung und die Sauerstoff und Kohlenstoff enthaltende Einsatzverbindung von einem Steamcrackingverfahren, das in den gemäß Ausgestaltungen der vorliegenden Erfindung vorgeschlagenen Kombinationen nicht oder allenfalls zusätzlich zu einem entsprechenden Olefinerzeugungsverfahren eingesetzt wird. Auch die ODH bzw. ODHE an sich stellt bereits ein (alternatives) Olefinerzeugungsverfahren dar. Wenn hier aber konkret von einem Olefinerzeugungsverfahren gesprochen wird, ist stets ein zusätzlich zur ODH bzw. ODHE durchgeführtes, andersartiges Olefinerzeugungsverfahren gemeint, das nicht auf einer oxidativen Dehydrierung wie der ODH bzw. ODHE beruht, sondern eben eine katalytischen Umsetzung einer Sauerstoff und Kohlenstoff enthaltenden Einsatzverbindung beinhaltet. Insbesondere handelt es sich dabei um die eingangs erwähnten Verfahren zur Umsetzung von Methanol zu Olefinen bzw. Propylen und auch um die Hydrierung von Kohlendioxid und die Herstellung und Umsetzung von Fischer-Tropsch-Produkten.

In Ausgestaltungen der Erfindung lassen sich in besonders vorteilhafter Weise insbesondere die spezifischen (höheren) Kohlendioxid-, Sauerstoff- und/oder Acetylengehalte im ersten Produktstrom, d.h. dem Produktstrom der ODHE, im Vergleich zum zweiten Produktstrom des Olefinerzeugungsverfahrens berücksichtigen. Ausgestaltungen der vorliegenden Erfindung betreffen dabei zunächst eine vorteilhafte Ausführung der Kohlendioxidentfernung und ergänzen diese bedarfsweise, und in weiteren vorteilhaften Ausgestaltungen, um weitere Elemente, insbesondere zur Sauerstoff- und/oder Acetylenentfernung.

Besonders relevant sind in Ausgestaltungen der Erfindung Aspekte zum Sauerstoffund Acetylengehalt im Produktstrom einer ODHE (hier also dem "ersten Produktstrom"). Dieser erste Produktstrom aus einer ODHE enthält dabei am Reaktoraustritt (hier also bezogen auf den feuchten Strom inklusive Wasser und Essigsäure) typischerweise mehr als 250 vol.-ppm (Millionstel Volumenanteile), mehr als 500 vol.-ppm, mehr als 1.000 vol.-ppm, mehr als 2.500 vol.-ppm, mehr als 5.000 vol.-ppm und teils bis zu mehr als 10.000 vol.-ppm Sauerstoff. Dies beruht auf der Tatsache, dass ODHE-Katalysatoren (insbesondere vom Typ MoVNbTeOₓ) nur mit einem gewissem Restgehalt an Sauerstoff betrieben werden, der insbesondere auch am Reaktoraustritt vorhanden ist. Nach Auskondensation von Wasser und Essigsäure erhöhen sich diese Werte in einem hierbei erhaltenen Folgestrom des ersten Produktstroms entsprechend auf mehr als 500 vol.-ppm, mehr als 1.000 vol.-ppm, mehr als 2.000 vol.-ppm, mehr als 5.000 vol.ppm, mehr als 10.000 vol.-ppm und teils bis zu mehr als 20.000 vol.-ppm Sauerstoff.

Der Acetylengehalt im (ersten) Produktstrom der ODHE am Reaktoraustritt eines ODHE-Reaktors (also ebenfalls im feuchten Prozessgas inklusive Essigsäure) beträgt typischerweise 50 vol.-ppm bis 2000 vol.-ppm, insbesondere 100 vol.-ppm bis 1.000 vol.-ppm, insbesondere 150 vol.-ppm bis 750 vol.-ppm, korrespondierend zu ca. 100 vol.-ppm bis 4.000 vol.-ppm, insbesondere 200 vol.-ppm bis 2.000 vol.-ppm, insbesondere 300 vol.-ppm bis 1.500 vol.-ppm nach einer entsprechenden Auskondensation von Wasser und Essigsäure in einem Folgestrom.

Die Gehalte an Kohlenmonoxid sowie an Kohlendioxid belaufen sich unabhängig voneinander im (ersten) Produktstrom der ODHE am Reaktoraustritt (also im feuchten Prozessgas inkl. Essigsäure) jeweils typischerweise auf 0,25 vol.-% (Volumenprozent) bis 5,0 vol.-% insbesondere 0,5 vol.-% bis 4,0 vol.-%, insbesondere 0,75 vol.-% bis 2,5 vol.-%, korrespondierend zu ca. 0,5 vol.-% bis 10,0 vol.-% insbesondere 1,0 vol.-% bis 8,0 vol.-%, insbesondere 1,5 vol.-% bis 5,0 vol.-% nach einer entsprechenden Auskondensation von Wasser und Essigsäure in einem Folgestrom.

In einer Ausgestaltung des vorgeschlagenen Verfahrens, die hier auch als Ausgestaltung A bezeichnet wird, umfassen der oder die dritten Reinigungs- und/oder Trennschritte eine Kohlendioxidentfernung, wobei die Bildung des ersten Folgestroms und des zweiten Folgestroms jedoch jeweils ohne eine Durchführung einer Kohlendioxidentfernung erfolgt.

In einer mit A bezeichneten Ausgestaltung erfolgt dabei nur im Zuge des oder der dritten Reinigungs- und/oder Trennschritte bzw. nur in einem dritten Reinigungs- und Zerlegungsteil, mittels dessen der oder die dritten Trennschritte implementiert werden, eine Laugewäsche. (Wie erwähnt ist bzw. sind dann, wenn von einem ersten, zweiten oder dritten "Reinigungs- und Zerlegungsteil" die Rede ist, auch der oder die hiermit realisierten ersten, zweiten oder dritten Reinigungs- und/oder Trennschritte gemeint.)

Die Ausgestaltung A eignet sich insbesondere für den Fall, dass die Kapazität der ODHE deutlich geringer als jene des Olefinerzeugungsverfahrens ist. Dies ist insbesondere dann der Fall, wenn die ODHE ausschließlich oder überwiegend mit Ethan aus dem Olefinerzeugungsverfahren gespeist wird, also keine oder nur geringe Zuführung von Ethan aus einer weiteren Quelle erfolgt.

Bei einer Kapazität des Olefinerzeugungsverfahrens von mehreren hundert Jahreskilotonnen Olefinen beträgt hier der anfallende Ethanstrom, der für eine ODHE genutzt wird, typischerweise weniger als 50 Jahreskilotonnen, 30 Jahreskilotonnen, 20 Jahreskilotonnen, 10 Jahreskilotonnen, 5 Jahreskilotonnen und insbesondere weniger als 2 Jahreskilotonnen. Somit ist die Ausführungsform A also besonders geeignet und vorteilhaft, wenn das Verhältnis des ersten Folgestroms (aus der ODHE) und des zweiten Folgestroms (aus dem Olefinerzeugungsverfahren) weniger als 1:3, weniger als 1:5, weniger als 1:10, weniger als 1:20 oder weniger als 1:50 beträgt.

Aufgrund der Tatsache, dass der erste Produktstrom bzw. dessen Folgestrom aus einer ODHE also eine deutlich geringere Menge umfasst als der zweite Produktstrom bzw. dessen Folgestrom aus dem Olefinerzeugungsverfahren, ist zunächst die sich ergebende Verdünnung bei Vereinigung der Produkt- bzw. Folgeströme ausreichend, so dass die Sauerstoffmenge aus der ODHE keine Auswirkung auf den dritten Reinigungs- und Zerlegungsteil sowie die dort gewonnen aufgereinigten Produktfraktionen hat. Acetylen wird ebenfalls so weit verdünnt, dass sich im Eintrittsstrom eines dritten Reinigungs- und Zerlegungsteils eine Konzentration ergibt, die durch eine im Rahmen eines dem Olefinerzeugungsverfahren zuzurechnenden Acetylenentfernungsschrittes (z.B. Selektivhydrierung) problemlos handhabbar ist. Dies ist besonders vorteilhaft im Rahmen eines Revamps, wo eine ODHE zur Verwertung einer Ethanfraktion aus dem Olefinerzeugungsverfahren nachgerüstet wird.

Es verbleibt somit lediglich die Aufgabe einer vorteilhaften Entfernung von Kohlendioxid. Dies kann in der hier aufgezeigten Konstellation durch eine Laugewäsche im dritten Reinigungs- und Zerlegungsteil erreicht werden. Mit anderen Worten kann die von dem oder den dritten Reinigungs- und/oder Trennschritten umfasste Kohlendioxidentfernung insbesondere in Form einer Laugewäsche durchgeführt werden. Eine vergleichsweiese aufwändige regenerative Kohlendixidentfernung ist also nicht erforderlich.

Es wird also wiederum der bereits erwähnte Verdünnungseffekt ausgenutzt. Überraschenderweise kann somit sowohl auf eine Rohgasbehandlung als auch auf eine eigene Kohlendioxidentfernung, insbesondere in Form einer Aminwäsche, im ersten Reinigungs- und Zerlegungsteil verzichtet werden, wie sie ansonsten bei einem ODHE-Verfahren typischerweise zwingend zum Einsatz kommen. Lediglich die Nutzung einer gemeinsamen Laugewäsche im dritten (gemeinsamen) Reinigungs- und Zerlegungsteil ist als vorteilhaftes Element vorgesehen.

Somit kann in entsprechenden Ausgestaltungen vorgesehen sein, dass, mit den erwähnten Vorteilen, die Bildung des ersten Teil- oder Folgestroms ohne eine Rohgasbehandlung und/oder Kohlendioxidentfernung durchgeführt wird.

Der Begriff "Rohgasbehandlung" bezeichnet eine Behandlung des (ersten) Produktstroms aus der oxidativen Dehydrierung, oder eines hiervon abgeleiteten Stromes, d.h. eines Folgestroms. Eine Rohgasbehandlung gemäß Ausgestaltungen der Erfindung zeichnet sich dadurch aus, dass Acetylen und Sauerstoff gleichzeitig aus dem Produktstrom bzw. dessen Folgestrom entfernt oder teilentfernt werden, wobei eine Abreicherung in gleichem Umfang oder in unterschiedlichen Umfängen erfolgen kann, beispielsweise um 100%, 90%, 80%, 70%, 60% oder 50%, bezogen auf den jeweils ursprünglich enthaltenen Anteil. Durch weitere Reaktion von Sauerstoff mit Kohlenmonoxid kann dabei auch eine partielle Verringerung des Gehalts an Kohlenmonoxid erfolgen, wobei zusätzliches Kohlendioxid gebildet wird. In der Rohgasbehandlung werden vorzugsweise ein oder mehrere, Kupferoxid enthaltende Katalysatoren oder ein oder mehrere, zumindest eines der Elemente Kupfer, Mangan, Zink, Nickel, Platin, Palladium, Rhodium und/oder Ruthenium enthaltende Katalysatoren, insbesondere Mischoxidkatalysatoren, verwendet.

Merkmale und Vorteile einer derartigen Rohgasbehandlung sind in der Patentliteratur, beispielsweise in der WO 2020/187572 A1, der EP 3 708 557 A1, der EP 3 708 558 A1 und der WO 2018/153831A1, beschrieben, wobei auf diese Druckschriften hier ausdrücklich Bezug genommen wird.

Bei einer ODHE bzw. in einer nachgelagerten Rohgasbehandlung gebildetes Kohlendioxid wird gemäß Stand der Technik üblicherweise durch eine der ODHE zugeordnete Aminwäsche sowie ggf. eine nachfolgende Laugewäsche als Feinreinigung entfernt, bevor eine weitere Aufreinigung und Auftrennung des Produktstromes bzw. eines entsprechenden Folgestroms in einem Zerlegungsteil erfolgt, der insbesondere an sich bekannte kryogene Verfahrensschritte (z.B. Demethanizer, Deethanizer, Splitter) umfassen kann.

In einer mit B bezeichneten Ausgestaltung der Erfindung erfolgt dagegen eine Rohgasbehandlung, der der erste Produktstrom zur Bildung des ersten Folgestroms unterworfen wird. Die Bildung des ersten Teil- oder Folgestroms umfasst hierbei also eine Rohgasbehandlung. Diese Ausgestaltung eignet sich insbesondere für Fälle, in denen eine Acetylenentfernung in der zur Ausgestaltung A erläuterten Weise in dem gemeinsamen, d.h. in dem dritten, Reinigungs- und Zerlegungsteil nicht mehr ausreichend ist und/oder der Sauerstoffgehalt einen zu hohen Wert erreicht, um bewältigt werden zu können.

Das Prozessgas aus einer ODHE enthält am Reaktoraustritt typischerweise, wie bereits oben erwähnt, Anteile an Sauerstoff und Acetylen. Nimmt nun die Menge des ersten Produktstromes bzw. dessen Folgestroms aus der ODHE im Vergleich zu dem zweiten Produktstrom bzw. dessen Folgestrom aus dem Olefinerzeugungsverfahren zu (z.B. bei zusätzlicher Einspeisung von Ethan aus anderer Quelle in die ODHE) und/oder ist der Verdünnungseffekt nicht mehr ausreichend, um einen gewünschten oder technisch notwendigen Acetylen- und/oder Sauerstoffgehalt an einer beliebigen Stellen in einem gemeinsamen, d.h. in dem dritten, Reinigungs- und Zerlegungsteil einzuhalten oder um eine erforderliche Produktspezifikation eines in diesem gemeinsamen, d.h. in dem dritten, Reinigungs- und Zerlegungsteil erhaltenen Zielproduktes zu erreichen, kommt zusätzlich zu Ausgestaltung A für den ersten Produktstrom bzw. einen Folgestrom hiervon an geeigneter Stelle eine Rohgasbehandlung zum Einsatz.

Im Vergleich zu einer eigenständigen, nicht integrierten ODHE-Anlage muss dabei nicht notwendigerweise eine vollständige Sauerstoff- und /oder Acetylenentfernung bei Bildung des Folgestroms des ersten Produktstroms erfolgen. Es kann weiterhin der zur Ausgestaltung A erläuterte Verdünnungseffekt vorteilhaft genutzt werden. Somit wird der Aufwand für die Rohgasbehandlung minimiert (apparativ und hinsichtlich der Erstellungskosten) sowie auch ein möglicher Verlust an Ethylen in der Rohgasbehandlung kann minimiert werden. Hierzu trägt insbesondere die Tatsache bei, dass eine Rohgasbehandlung nicht "scharf" gefahren werden muss, also auch jederzeit in einem milden Bereich gefahren werden kann. Dies erhöht zudem die Standzeit einer Rohgasbehandlung.

Gemäß einer Ausgestaltung der Erfindung beträgt ein Restsauerstoffgehalt des ersten Produktstroms nach der Rohgasbehandlung und damit eines entsprechenden Folgestroms des ersten Produktstroms weniger als 100 vol.-ppm, 50 vol.-ppm, 25 vol.-ppm, 10 vol.-ppm, 5 vol.-ppm, 2 vol.-ppm, 1 vol.-ppm, 0,5 vol.-ppm oder 0,1 vol.-ppm.

Gemäß einer Ausgestaltung der Erfindung beträgt ein Restacetylengehalt des ersten Produktstroms nach der Rohgasbehandlung und damit des Folgestroms des ersten Produktstroms weniger als 100 vol.-ppm, 50 vol.-ppm, 25 vol.-ppm, 10 vol.-ppm, 5 vol.-ppm, 2 vol.-ppm, 1 vol.-ppm, 0,5 vol.-ppm, 0,3 vol.-ppm oder 0,1 vol.-ppm.

In den Ausführungsformen A und B kann der erste Reinigungs- und Zerlegungsteil bedarfsweise zumindest eine Verdichtung umfassen, so dass die Folgeströme des ersten und zweiten Produktstroms insbesondere auf gleichem Druckniveau vereinigt werden können. Die Bildung des ersten Teil- oder Folgestroms kann also eine Druckerhöhung umfassen.

Sofern der zweite Produktstrom bzw. dessen Folgestrom aus dem Olefinerzeugungsverfahren auf ähnlich niedrigem Niveau wie der erste Produktstrom aus der ODHE bereitgestellt werden, kann eine solche Verdichtung im ersten Reinigungs- und Zerlegungsteil jedoch auch entfallen und eine Verdichtung erst im dritten Reinigungs- und Zerlegungsteil erfolgen. Üblicherweise wird ein Olefinerzeugungsverfahren jedoch auf einem höheren Druckniveau als die ODHE durchgeführt und der erste Reinigungs- und Zerlegungsteil umfasst daher vorzugsweise eine Verdichtung.

Zudem wird eine Kohlendioxidentfernung grundsätzlich bevorzugt auf einem möglichst hohen Druckniveau durchgeführt.

In einer mit C bezeichneten Ausgestaltung erfolgt auch eine Kohlendioxidentfernung separat für den ersten Produktstrom bzw. einen Folgestrom hiervon. Die Bildung des ersten Teil- oder Folgestroms kann also eine (Vor-)Entfernung von Kohlendioxid umfassen. Diese Vorentfernung erfolgt insbesondere als "Bulk-Removal", d.h. als eine Grobentfernung eines wesentlichen Anteils.

Ist eine im dritten Reinigungs- und Zerlegungsteil vorhandene Laugewäsche nicht (mehr) ausreichend, um die gesamte Kohlendioxidfracht aus dem Olefinerzeugungsverfahren und insbesondere der ODHE zu gewährleisten, so kann der der ODHE zugeordnete Reinigungs- und Zerlegungsteil eine Kohlendioxidentfernung beinhalten, die eine Vorentfernung oder "Bulk-Removal" von Kohlendioxid aus dem Produktstrom der ODHE bewirkt. Hierzu ist zunächst grundsätzlich eine bereits zuvor erwähnte Aminwäsche geeignet, jedoch kann auch eine weiter unten zu Ausgestaltung F beschriebene Variante zum Einsatz kommen.

Eine Ausführung mit und ohne Berücksichtigung einer Rohgasbehandlung, wie zu Ausgestaltung B beschrieben, ist entsprechend den jeweiligen Erfordernissen möglich. Eine angepasste Entfernung von Kohlendioxid einerseits sowie andererseits Sauerstoff und Acetylen ist also bedarfsgerecht und unabhängig voneinander möglich, um also weiterhin eine größtmögliche Effizienzsteigerung bei der Integration von ODHE und Olefinerzeugungsverfahren zu erreichen.

In dieser Ausführungsform C kann insbesondere der zunächst in der ODHE im Vergleich zum Olefinerzeugungsverfahren anfallende hohe Kohlendioxidgehalt und auch der in einer Rohgasbehandlung wie zu Ausgestaltung B beschrieben entstehende zusätzliche Kohlendioxidanteil reduziert bzw. weitestgehend entfernt werden. Insbesondere bei Olefinerzeugungsverfahren, bei denen kein oder nur wenig Kohlendioxid im zweiten Produktgemisch bzw. einem Folgegemisch hiervon enthalten ist, dient dann also die Laugewäsche im gemeinsamen Reinigungs- und Zerlegungsteil zur Feinreinigung und kann entsprechend klein dimensioniert werden. Im Falle eines Revamps, also insbesondere der Nachrüstung einer ODHE in Ergänzung zu einem bestehenden Olefinerzeugungsverfahren, kann vorteilhafterweise eine bereits vorhandene Laugewäsche ausreichend sein.

Gemäß dieser Ausgestaltung der Erfindung beträgt ein Restkohlendioxidgehalt nach der Kohlendioxidentfernung in dem der ODHE zugeordneten, d.h. in dem ersten, Reinigungs- und Zerlegungsteil und/oder eines entsprechenden Folgestroms hiervon weniger als 1,0 vol.-%, 0,5 vol.-%, 0,25 vol.-% oder 0,1 vol.-%.

In einer mit D bezeichneten Ausgestaltung erfolgt eine Aminwäsche im gemeinsamen Reinigungs- und Zerlegungsteil. Die von dem oder den dritten Reinigungs- und/oder Trennschritten umfasste Kohlendioxidentfernung kann also eine Aminwäsche umfassen. Diese Variante eignet sich insbesondere dann, wennn es sich beim zweiten Produktstrom aus dem Olefinerzeugungsverfahren bzw. einem Folgestrom hiervon ebenfalls um einen kohlendioxidreichen Strom handelt, wie es insbesondere bei der Umsetzung von kohlendioxidhaltigen Einsatzströmen im Olefinerzeugungsverfahren zu erwarten ist. In dieser Ausgestaltung umfasst eine Kohlendioxidentfernung im gemeinsamen Reinigungs- und Zerlegungsteils insbesondere eine Aminwäsche, wohingegen eine eigenständige Aminwäsche im Reinigungs- und Zerlegungsteil, der der ODHE zugeordnet ist, d.h. dem ersten Reinigungs- und Zerlegungsteil, insbesondere nicht vorgesehen ist.

Eine Doppelung dieser Schritte zur Kohlendioxidentfernung kann somit vermieden werden. Sowohl die Aminwäsche als auch eine Laugewäsche zur Feinreinigung werden nur im gemeinsamen Reinigungs- und Zerlegungsteil im Rahmen einer hier erfolgenden Kohlendioxidentfernung benötigt.

Eine hier mit E bezeichnete Ausgestaltung entfaltet spezielle Vorteile durch eine Integration von Prozesswasser aus dem Olefinerzeugungsverfahren und der Entfernung von Kohlendioxid im Reinigungs- und Zerlegungsteil der ODHE. In dem einen oder den mehreren katalytischen Umsetzungsschritten anfallendes Prozesswasser kann also in einer solchen Ausgestaltung bei der Bildung des zweiten Teil- oder Folgestroms verwendet werden.

In dieser Ausführungsform ergibt sich ein zusätzlicher Vorteil durch Nutzung des in dem Olefinerzeugungsverfahren gebildeten Wassers. Nach der Auskondensation und Abtrennung von Wasser und Essigsäure aus dem ersten Produktstrom in einer Kondensatabscheidung kann der Gasstrom immer noch Spuren von Essigsäure enthalten. Um diese zu entfernen kann in dem der ODHE zugeordneten Reinigungsund Zerlegungsteil d.h. dem ersten Reinigungs- und Zerlegungsteil, ein weiterer Absorber (Wäscher) eingesetzt werden, der mit einem wässrigen Kondensat aus dem Olefinerzeugungsverfahren gespeist wird. Da das Reaktionsgas der ODHE bei Eintritt in diesen Wäscher nur noch Spuren von Essigsäure enthält, stellt sich im Wäscher ein neutraler bzw. nur leicht saurer pH-Wert ein und es kann grundsätzlich auch ein gewisser Anteil von Kohlendioxid aus dem Reaktionsgas entfernt werden. Der Austrittstrom aus dem Wäscher ist also frei oder weitestgehend frei an Essigsäure und kann abgereichert sein an Kohlendioxid. Allerdings ist der Effekt der Kohlendioxidentfernung hier relativ gering ausgeprägt, d.h. als wesentlicher Vorteil ergibt sich die Entfernung von Essigsäure, was vorteilhaft in Bezug auf die Materialauswahl in folgenden Anlagenteilen ist.

In einer mit F bezeichneten Ausgestaltung ergeben sich spezielle Vorteile durch eine Nutzung der Ablauge aus einer Laugewäsche im dritten Reinigungs- und Zerlegungsteil. Eine Ablauge aus der von dem oder den dritten Reinigungs- und/oder Trennschritten umfassten Kohlendioxidentfernung kann also bei der Bildung des ersten Teil- oder Folgestroms verwendet werden.

Um eine signifikante Verringerung der Kohlendioxidkonzentration im Reinigungs- und Zerlegungsteil der ODHE, d.h. dem ersten Reinigungs- und Zerlegungsteil, zu erreichen, kann besonders vorteilhaft ganz oder anteilig die Ablauge aus einer Laugewäsche im gemeinsamen Reinigungs- und Zerlegungsteil d.h. dem dritten Reinigungs- und Zerlegungsteil in einem weiteren Adsorber verwendet werden. Da der erste Produkt- und dessen Folgestrom der ODHE eine vergleichsweise hohe Kohlendioxidkonzentration aufweist, die üblicherweise und insbesondere in dieser Ausführungsform höher ist als die Kohlendioxidkonzentration im zweiten Produkt- und dessen Folgestrom und somit auch in dem aus den Folgeströmen gebildeten Eintrittsstrom in den gemeinsamen Reinigungs- und Zerlegungsteil, d.h. dem dritten Reinigungs- und Zerlegungsteil, verfügt die Ablauge immer noch über eine gewisse Restkapazität, um im Produktstrom der ODHE Kohlendioxid abzureichern.

Insbesondere kann auf diese Weise im ersten Reinigungs- und Zerlegungsteil die Kohlendioxidkonzentration um einen Faktor von bis zu 0,9, bis zu 0,8, bis zu 0,7, bis zu 0,6, bis zu 0,5 und bis zu 0,3 verringert werden.

Eine Kombination der Ausführungsform F mit den zuvor beschriebenen Ausführungsformen B, D und E ist ebenfalls Gegenstand von Ausgestaltungen der vorliegenden Erfindung. Insbesondere stellt die Ausführungsform F eine besonders vorteilhafte Gestaltung der Ausführungsform C dar. Es ergeben sich dabei insbesondere Vorteile in Bezug auf die Nutzung von Betriebsmitteln.

Die vorgeschlagene Anlage zur Herstellung eines oder mehrerer Syntheseprodukte ist dafür eingerichtet, Ethan als eine erste Einsatzverbindung in einem ersten Einsatzstrom unter Erhalt eines Ethylen als ein erstes Produktolefin enthaltenden ersten Produktstroms einer oxidativen Dehydrierung zu unterwerfen, und eine Sauerstoff und Kohlenstoff enthaltende zweite Einsatzverbindung in einem zweiten Einsatzstrom unter Erhalt eines das erste und/oder ein zweites Produktolefin enthaltenden zweiten Produktstroms einem oder mehreren katalytischen Umsetzungsschritten zu unterwerfen.

Die vorgeschlagene Anlage weist Mittel auf, die dafür eingerichtet sind, zumindest einen Teil des ersten Produktstroms unter Erhalt eines ersten Folgestroms einem oder mehreren ersten Reinigungs- und/oder Trennschritten zu unterwerfen, zumindest einen Teil des zweiten Produktstroms unter Erhalt eines zweiten Folgestroms einem oder mehreren zweiten Reinigungs- und/oder Trennschritten zu unterwerfen, zumindest einen Teil des ersten Folgestroms und zumindest einen Teil des zweiten Folgestroms zur Bildung eines dritten Einsatzstroms zu verwenden und den dritte Einsatzstrom einem oder mehreren dritten Reinigungs- und/oder Trennschritten zuzuführen.

Zu weiteren Merkmalen und Vorteilen einer entsprechenden Anlage und Ausgestaltungen hiervon sei auf die obigen Erläuterungen betreffend das erfindungsgemäß vorgeschlagene Verfahren und seine Ausgestaltungen ausdrücklich verwiesen, da diese hierfür in gleicher Weise gelten.

Entsprechendes gilt auch für eine Anlage, die gemäß einer Ausgestaltung der Erfindung dazu eingerichtet ist, ein Verfahren gemäß einer beliebigen Ausgestaltung der vorliegenden Erfindung durchzuführen.

### Kurze Beschreibung der Zeichnung

Ausführungsformen der Erfindung werden nachfolgend rein beispielhaft unter Bezugnahme auf die beigefügte Zeichnung beschrieben, wobei
- Figur 1: ein Verfahren gemäß einer Ausgestaltung veranschaulicht, und
- Figur 2: Aspekte eines Verfahrens gemäß einer Ausgestaltung veranschaulicht.

### Ausführungsformen der Erfindung

Die nachfolgend beschriebenen Ausführungsformen werden lediglich zu dem Zweck beschrieben, den Leser beim Verständnis der beanspruchten und zuvor erläuterten Merkmale zu unterstützen. Sie stellen lediglich repräsentative Beispiele dar und sollen hinsichtlich der Merkmale der Erfindung nicht abschließend und/oder beschränkend betrachtet werden. Es versteht sich, dass die zuvor und nachfolgend beschriebenen Vorteile, Ausführungsformen, Beispiele, Funktionen, Merkmale, Strukturen und/oder anderen Aspekte nicht als Beschränkungen des Umfangs der Erfindung, wie er in den Ansprüchen definiert ist, oder als Beschränkungen von Äquivalenten zu den Ansprüchen zu betrachten sind, und dass andere Ausführungsformen verwendet und Änderungen vorgenommen werden können, ohne vom Umfang der beanspruchten Erfindung abzuweichen.

Unterschiedliche Ausführungsformen der Erfindung können weitere zweckmäßige Kombinationen der beschriebenen Elemente, Komponenten, Merkmale, Teile, Schritte, Mittel usw. umfassen, aufweisen, aus ihnen bestehen oder im Wesentlichen aus ihnen bestehen, auch wenn solche Kombinationen hier nicht spezifisch beschrieben sind. Darüber hinaus kann die Offenbarung andere Erfindungen umfassen, die gegenwärtig nicht beansprucht sind, die aber in Zukunft beansprucht werden können, insbesondere wenn sie vom Umfang der unabhängigen Ansprüche umfasst sind.

Erläuterungen, die sich auf Vorrichtungen, Apparate, Anordnungen, Systeme usw. gemäß Ausführungsformen der vorliegenden Erfindung beziehen, können auch für Verfahren, Prozesse, Methoden usw. gemäß den Ausführungsformen der vorliegenden Erfindung gelten und umgekehrt. Gleiche, gleich wirkende, in ihrer Funktion einander entsprechende, baulich identisch oder vergleichbar aufgebaute Elemente, Verfahrensschritte usw. können mit identischen Bezugszeichen angegeben sein.

Die vorliegende Erfindung und ihre Ausgestaltungen gehen, wie mehrfach erwähnt, zunächst von der ODH, insbesondere der ODHE, aus. Diese wird in Ausgestaltungen der Erfindung mit einem oder mehreren der ebenfalls eingangs erwähnten Olefinerzeugungsverfahren kombiniert, d.h. insbesondere mit einem oder mehreren
1. MTO- bzw. MTP-Verfahren ausgehend von Methanol und/oder Dimethylether,
2. Fischer-Tropsch-Verfahren unter Erhalt von Olefinen, d.h. FTTO, und/oder
3. direkter Umsetzung von Kohlendioxid bzw. kohlendioxidhaltigem Synthesegas.

Diese Olefinerzeugungsverfahren umfassen, wie ebenfalls erwähnt, als Gemeinsamkeit einen oder mehrere katalytische Umsetzungsschritte, dem bzw. denen eine Sauerstoff und Kohlenstoff enthaltende Einsatzverbindung zugeführt wird.

Während die der ODH bzw. ODHE zugeführte Einsatzverbindung, insbesondere Ethan, als "erste Einsatzverbindung" bezeichnet wird, wird die Sauerstoff und Kohlenstoff enthaltende Einsatzverbindung, die dem einen oder den mehreren katalytischen Umsetzungsschritten zugeführt wird, hier auch als "zweite" Einsatzverbindung bezeichnet. Bei der zweiten Einsatzverbindung kann es sich um Kohlenmonoxid, Kohlendioxid oder ein Oxygenat wie Methanol oder Dimethylether handeln.

Nachfolgend sollen zunächst weitere Aspekte entsprechender Verfahren erläutert werden, um den Hintergrund der Erfindung und ihrer Ausgestaltungen zu veranschaulichen, bevor auf konkrete Ausgestaltungen Bezug genommen werden. Die ODH bzw. ODHE ist, wie eingangs erwähnt, an sich bekannt, wobei nachfolgend insbesondere die ODHE erläutert wird, die entsprechenden Erläuterungen aber auch für die ODH anderer Kohlenwasserstoffe außer Ethan sinngemäß gelten.

An Olefinerzeugungsverfahren sind, wie erwähnt, zunächst das MTO- und als Variante davon das MTP-Verfahren wohlbekannt und technisch etabliert. Bei diesen Verfahren wird Methanol an geeigneten Katalysatoren (typischerweise Molekularsiebe oder Zeolithe) zu einem Gemisch von Kohlenwasserstoffen unterschiedlicher Kettenlänge, insbesondere Paraffinen und Olefinen, umgesetzt. Andere bekannte Varianten gehen auch von Dimethylether als Intermediat anstelle von Methanol, oder auch Mischungen von Dimethylether und Methanol, aus.

Als weiteres Reaktionsprodukt entsteht bei MTO- und MTP-Verfahren Wasser. Acetylen entsteht bei MTO- und MTP-Verfahren üblicherweise nur in sehr geringen Anteilen, und die entsprechenden Verfahren enthalten demzufolge nur eine für solch geringe Mengen ausgelegte Acetylenentfernung. Auch Kohlendioxid entsteht allenfalls nur in geringen Mengen von üblicherweise weniger als 0,5 vol.-%, weniger als 0,25 vol.-% und insbesondere weniger als 0,1 vol.-%, die sich vergleichsweise einfach mittels einer Laugewäsche entfernen lassen. Es kann beispielsweise auf Fachliteratur wie einen Artikel von M.R. Gogate, "Methanol-to-olefins process technology: current status and future prospects", Petroleum Science and Technology 2019, 37 (5), 559-565, verwiesen werden, in welchem eine aktuelle Übersicht über entsprechende technische MTO- bzw. MTP-Verfahren inklusive typischer Verfahrensschemata enthalten ist. Wesentliche Prozessschritte des Zerlegungsteils einer MTO- bzw. MTP-Anlage können dabei Wasserabtrennung, Laugewäsche, Trocknung, Verdichtung, Demethanisierung, Deethanisierung, Depropanisierung sowie Trennung von Kohlenwasserstoffen mit zwei Kohlenstoffatomen und Trennung von Kohlenwasserstoffen mit drei Kohlenstoffatomen voneinander umfassen. Bekannt sind ebenfalls Verfahren zur Herstellung von benzinartigen Verbindungen aus Methanol (engl. Methanol to Gasoline, MTG), die jedoch typischerweise zu einem schweren Produktspektrum führen.

Das Fischer-Tropsch-Verfahren ist bekanntermaßen ein heterogen katalysiertes Verfahren zur Umwandlung von Synthesegas in flüssige Kohlenwasserstoffe. Grundlagen sind beispielsweise bei A. Steynberg und M. Dry (Hrsg.), "Fischer-Tropsch Technology", Elsevier, 2006, Bd. 152, oder A. de Klerk, "Small-Scale Fischer-Tropsch gas-to-liquids facilities: advances and applications", In: Davis, B.H. und Occelli, M.L. (Hrsg.), "Fischer-Tropsch Synthesis, Catalysts, and Catalysis", CRC Press, 2016 zu finden. Die Produkte können je nach den Prozessbedingungen von kurzen bis langen Kohlenwasserstoffketten variieren und Paraffine sowie Olefine umfassen. Zumeist werden Eisen- oder Cobaltkatalysatoren zwischen 150 und 300 °C eingesetzt. Die Selektivität von Fischer-Tropsch-Verfahren kann durch die Betriebsbedingungen, die Art des Katalysators, den Träger und die Reaktorkonfiguration eingestellt werden. Neuere Ansätze verfolgen insbesondere auch eine gesteigerte Selektivität hin zu leichten Olefinen, siehe z.B. H.M. Torres Galvis et al., "Supported Iron Nanoparticles as Catalysts for Sustainable Production of Lower Olefins", Science, 335, 835-838, oder H.M. Torres Galvis et al., "Catalysts for Production of Lower Olefins from Synthesis Gas: A Review", ACS Catal., 3, 2130-2149.

Vergleichbar zu den o.g. MTO- bzw. MTP-Verfahren erfordert die breite Produktverteilung, die Olefine und Paraffine umfasst, eine geeignete Fraktionierung, die die bereits bei MTO und MTP erwähnten Prozessschritte eines Zerlegungsteils umfassen kann. Die Acetylen- und Kohlendioxidgehalte belaufen sich dabei in etwa auf die gleiche Größenordnung wie bei den zuvor genannten MTO- und MTP-Verfahren.

Die direkte Umsetzung von Kohlendioxid bzw. kohlendioxidhaltigem Synthesegas gewinnt insbesondere im Rahmen von Bestrebungen zur Verringerung von Kohlendioxidemissionen an Bedeutung. Aktuelle Forschungsrichtungen zu diesem Ansatz zielen auf die Umsetzung von Kohlendioxid mit Wasserstoff (ggf. auch in Anwesenheit von Kohlenmonoxid) hin zu Kohlenwasserstoffen ab. Im Fokus liegen dabei insbesondere Produkte mit zwei bis vier Kohlenstoffatomen (sowohl Paraffine als auch Olefine), insbesondere Propylen. Auch Mischungen von Kohlendioxid und Kohlenmonoxid können dabei zum Einsatz kommen, ein entsprechender Reaktionsschritt wird also mit einer Mischung von Kohlendioxid und Wasserstoff bzw. mit einem kohlendioxidreichen Synthesegas gespeist.

In der Fachliteratur ist beispielsweise auch eine Kombination aus Reforming und Fischer-Tropsch- bzw. FTTO-Verfahren zu Olefinen beschrieben. Ferner finden sich in dort auch bereits Ansätze, um eine Methanolsynthese aus Synthesegas und MTO- bzw. MTP-Verfahren in einem Schritt zu kombinieren. Diese Ansätze basieren üblicherweise auf einer Kombination von Zirkon-Zink-Oxiden (zur Methanolsynthese) und H-SAPO-34 als MTO- bzw. MTP-Katalysator.

Auch existieren bereits Entwicklungen zur Hydrierung von Kohlendioxid über bifunktionale Katalysatoren (hierzu beispielsweise W. Li et al., "A short review of recent advances in CO2 hydrogenation to hydrocarbons over heterogeneous catalysts", RSC Adv., 2018, 8, 7651, K. Cheng, et al., "Direct and Highly Selective Conversion of Synthesis Gas into Lower Olefins: Design of a Bifunctional Catalyst Combining Methanol Synthesis and Carbon-Carbon Coupling", Angew. Chem. Int. Ed. 2016, 55, 4725-4728 und F. Jiao et al., "Selective conversion of syngas to light olefins", Science 2016, 351, 1065-1068 und U. Olsbye, "Single-Pass Catalytic Conversion of Syngas into Olefins via Methanol", Angew. Chem. Int. Ed. 2016, 55, 7294 - 7295.

Grundsätzlich erscheinen derartige Systeme aber auch für die entsprechende Umsetzung von Kohlenmonoxid bzw. beliebiger Gemische aus Kohlenmonoxid und Kohlendioxid zu Kohlenwasserstoffen und insbesondere zu Olefinen wie Ethylen und/oder Propylen geeignet.

Die bifunktionalen Katalysatoren katalysieren dabei zwei Reaktionsschritte, nämlich eine Umsetzung der Komponenten von Synthesegas zu einem oder mehreren Oxygenaten wie Methanol und/oder Dimethylether als Intermediat(en) und die weitere Umsetzung des oder der Intermediate zur gewünschten Zielverbindung in Form des einen oder der mehreren Kohlenwasserstoffe. Ein hierbei verwendeter bifunktionaler Katalysator kombiniert typischerweise verwendete Methanolkatalysatoren mit aciden Zeolithstrukturen, welche die Folgereaktion katalysieren.

Alternativ kann der parallele Ablauf beider Reaktionsschritte aber auch durch eine Kombination von zwei oder mehreren geeigneten Katalysatoren, die jeweils jeder für sich bevorzugt die entsprechenden Reaktionsschritte katalysieren, als physikalische Mischung in einem Katalysatorbett erfolgen. Bei entsprechenden Katalysatoren kann es sich um die dem Grunde nach bekannten, zuvor genannten Katalysatoren zur Methanol- bzw. Dimethylethersynthese bzw. zur weiteren Umsetzung handeln. Entsprechend der mechanistischen Verwandtschaft des zweiten Reaktionsschrittes zu den zuvor genannten MTO- und MTP-Verfahren ist mit vergleichbaren Acetylengehalten im entsprechenden Produktstrom zu rechnen, verfahrensbedingt liegt der Kohlendioxidgehalt im Produktstrom eines solchen Kohlendioxid-basierten Verfahrens jedoch typischerweise deutlich höher und insbesondere mehr als 1 vol.-%, mehr als 5 vol. %, mehr als 10 vol.-% und kann auch mehr als 25 vol.-% betragen, sofern die Reaktion nur im Teilumsatz pro Durchgang gefahren wird.

Alle der genannten Olefinerzeugungsverfahren führen also nicht nur zu einem einzigen gewünschten Zielprodukt, sondern erzeugen immer ein Gemisch von Produkten, also Kohlenwasserstoffe unterschiedlicher Kettenlänge und mit unterschiedlichen Paraffinund Olefinanteilen. Es sind also immer wesentliche Anteile anderer Komponenten als nur einem einzigen gewünschten Zielprodukt enthalten.

Dies erfordert, wie beschrieben, eine geeignete Fraktionierung. Dabei weist die Fraktion mit einer bestimmten Anzahl von Kohlenstoffatomen insbesondere immer eine Mischung der entsprechenden Olefine und Paraffine auf, im Falle von zwei Kohlenstoffatomen sind dies Ethan und Ethylen. In bestimmten Ausführungsformen ist zwar nahezu eine selektive Erzeugung von Paraffinen bekannt (insbesondere bei Fischer-Tropsch und der Umsetzung von Kohlendioxid bzw. kohlendioxidhaltigem Synthesegas), solche Ausführungsformen sind im Rahmen der vorliegenden Erfindung jedoch weniger relevant und werden im Folgenden nicht weiter berücksichtigt.

Es ist im Rahmen der Olefinerzeugungsverfahren also zunächst immer nur die Maximierung einer Fraktion mit bestimmter Anzahl von Kohlenstoffatomen sowie eine Optimierung der Selektivität zu z.B. Olefinen möglich, jedoch nicht die ausschließliche oder nahezu ausschließliche Erzeugung eines einzigen Produktes oder nur von Olefinen. Dies gilt insbesondere für das im Rahmen von Ausgestaltungen der vorliegenden Erfindung relevante Zielprodukt Ethylen und damit auch das bei den erfindungsgemäßen Olefinerzeugungsverfahren in einer Fraktion mit Verbindungen mit zwei Kohlenstoffatomen zwangsläufig anfallende Ethan.

Acetylen ist typischerweise nicht oder eben nur in geringen Mengen im Produktstrom eines Olefinerzeugungsverfahrens der hier beschriebenen Art vorhanden. Hierunter ist insbesondere ein Acetylengehalt, der geringer ist als die oben genannten Acetylengehalte im Produktstrom einer ODHE, zu verstehen. Insbesondere bei MTO- bzw. MTP- und FTTO-Verfahren ist der Kohlendioxidgehalt im zweiten Produktstrom bzw. in einem Folgestrom hiervon kleiner als im ersten Produktstrom bzw. einem Folgestrom hiervon. Typische Verhältnisse im Kohlendioxidgehalt des entsprechenden ersten zum entsprechenden zweiten Produktstrom bzw. entsprechenden Folgeströmen (wie weiter unten definiert) betragen mehr als 4:1, mehr als 8:1, mehr als 12:1, mehr als 20:1 sowie mehr als 30:1.

Grundlegende Ansätze zu einer Integration von ODHE und MTO finden sich in den Patentanmeldungen CN 113845401 A, CN 113869985 A und CN 113896608 A. Diesen Schriften ist gemeinsam, dass eine Ethanfraktion aus einem MTO-Verfahren als Einsatzstoff für eine ODHE verwendet wird. Im Weiteren wird der Produktstrom einer ODHE nach einer absorbtiven Kohlendioxidentfernung in den Zerlegungsteil des MTO-Verfahrens eingespeist und dort mit dem Rohprodukt aus der MTO-Reaktion vereinigt. Auch ein Schritt zur Entfernung von Sauerstoff im Produktstrom der ODHE wird in diesen Schriften offenbart, diese Sauerstoffentfernung erfolgt jedoch ausschließlich durch Umsetzung von Sauerstoff mit Kohlenmonoxid und diese ist typischerweise stromauf einer Verdichtung angeordnet (vgl. CN 113896608 A). Auch eine Einspeisung eines Hilfsgases (insbesondere Kohlenmonoxid) für diese Sauerstoffentfernung ist Teil dieser Schriften. Es finden sich keinerlei Ausführungen zu einer geeigneten Entfernung von Acetylen aus dem Produktstrom einer ODHE und an keiner Stelle der Schriften wird auf die Tatsache eingegangen, dass der Produktstrom einer ODHE auch im Vergleich zu den Olefinerzeugungsverfahren erhebliche Anteile an Acetylen enthält. Wie bereits erwähnt, enthält aber der Zerlegungsteil von Olefinerzeugungsverfahren keine geeigneten Einrichtungen zur Acetylenentfernung von solch hohen Konzentrationen, wie sie typischerweise im Produktstrom einer ODHE auftreten. Somit offenbaren die Schriften keine geeignete Lösung zur Entfernung von Acetylen bei der Integration von Olefinerzeugungsverfahren und einer ODHE bei Verwendung eines gemeinsamen Zerlegungsteils.

Eine weitere Anmeldung, CN 113831207 A, umfasst die Verwendung einer Druckwechseladsorption (engl. Pressure Swing Adsorption, PSA) im Produktgasstrom einer ODHE zur Abtrennung von Verdünnungsgas und anderen (leichten) Komponenten wie Sauerstoff und Kohlenmonoxid vor der Einspeisung einer so gebildeten, an Kohlenwasserstoffen mit zwei Kohlenstoffatomen reichen Fraktion in den Zerlegungsteil eines MTO-Verfahrens. Auch dies stellt keine Lösung zu einer geeigneten Acetylenentfernung dar.

Für den Stand der Technik zu üblichen Verfahrensschritten wie Destillationen (kryogen und nicht-kryogen), Kohlendioxidentfernung etc. wird auf Fachliteratur bzw. dem Fachmann bekanntes Wissen verwiesen.

Wie bereits dargelegt enthält der zweite Produktstrom aus einem Olefinerzeugungsverfahren üblicherweise keinen Sauerstoff und im Vergleich zur ODHE nur geringe Anteile an Acetylen, ein entsprechender Zerlegungsteil eines Olefinerzeugungsverfahrens enthält demzufolge auch keine geeigneten Einrichtungen zur Entfernung von Sauerstoff und keine Einrichtungen zur Entfernung größerer Konzentrationen an Acetylen.

Eine solche Entfernung ist jedoch zwingend erforderlich in Hinblick auf Produktspezifikationen, insbesondere eines Ethylenproduktes, sowie auch aus verfahrens- und sicherheitstechnischen Gründen, da es ansonsten zu einer kritischen Anreicherung von Sauerstoff (Explosionsgefahr) oder Foulingeffekten kommen kann. Eine direkte Einspeisung eines ersten Produktstromes aus einer ODHE in den Zerlegungsteil eines Olefinerzeugungsverfahrens ist also gemäß Stand der Technik in der Regel nicht möglich. Die zuvor benannten Schriften lösen lediglich die Sauerstoffproblematik und erfordern für eine technische Umsetzung zwingend eine Lösung zum Umgang mit dem vergleichsweise hohen Acetylengehalt im Produktstrom einer ODHE.

Insbesondere ist darüber hinaus aber insbesondere eine vorteilhafte Lösung unter Berücksichtigung abweichender Kohlendioxidgehalte in einem ersten Produktstrom aus einer ODHE und einem zweiten Produktstrom aus einem Olefinerzeugungsverfahren notwendig, um ein effizientes Gesamtverfahren zu erreichen. Entsprechende Lösungen bieten die vorliegende Erfindung und ihre Ausgestaltungen.

Die Erfindung und ihre Ausgestaltungen lösen, wie zuvor bereits erwähnt, die genannten Probleme durch die Verknüpfung eines Olefinerzeugungsverfahrens mit einer ODHE, wobei überraschenderweise signifikante Vorteile in der Gestaltung des Zerlegungsteils erzielt werden können.

Dabei gilt es insbesondere die spezifischen (höheren) Kohlendioxid-, Sauerstoffund/oder Acetylengehalte in einem ersten Produktstrom einer ODHE im Vergleich zu einem zweiten Produktstrom eines Olefinerzeugungsverfahrens zu berücksichtigen. Ausgestaltungen der vorliegenden Erfindung gehen dabei zunächst auf eine vorteilhafte Ausführung der Kohlendioxidentfernung ein und ergänzen diese bedarfsweise um weitere Elemente zur Sauerstoff- und/oder Acetylenentfernung.

Zuvor und im Folgenden wird der Begriff "Reinigungs- und Zerlegungsteil" verwendet. Dieser kann insbesondere zur Durchführung eines oder mehrerer Verfahrensschritte eingerichtet sein bzw. verwendet werden, der oder die, in geeigneter Kombination, ausgewählt ist oder sind aus:
- Verdichtung
- Kondensation
- Abscheidung/Phasentrennung, insbesondere Gas-Flüssig-Trennung
- Trocknung
- destillativen bzw. rektifikatorischen Trennungen (kryogen und nicht-kryogen), insbesondere Abtrennung von Kohlenwasserstoffen mit zwei und mehr Kohlenstoffatomen von leichteren Verbindungen (Demethanisierung), Abtrennung von Kohlenwasserstoffen mit drei und mehr Kohlenstoffatomen von leichteren Verbindungen (Deethanisierung), Abtrennung von Kohlenwasserstoffen mit vier und mehr Kohlenstoffatomen von leichteren Verbindungen (Depropanisierung), Trennung (Split) zwischen Kohlenwasserstoffen mit zwei Kohlenstoffatomen, Trennung (Split) zwischen Kohlenwasserstoffen mit drei Kohlenstoffatomen, etc.
- Absorptive Entfernung von Komponenten, insbesondere mittels regenerierbarer und nicht regenerierbarer Wäschen (z.B. Kohlendioxidentfernung mittels Aminwäsche bzw. Laugewäsche)
- Rohgasbehandlung für das ODHE-Prozessgas wie bereits zuvor definiert
- Adsorptive Entfernung von Komponenten, insbesondere von Oxygenaten
- Druckwechseladsorption (engl Pressure Swing Adsorption, PSA)
- Membranabtrennungen
- Reaktive Entfernung von Komponenten, insbesondere mittels katalytischer Umsetzung, z.B. Hydrierungen und/oder Oxidationen

Weitere relevante Verfahrensschritte können eine Essigsäureaufbereitung umfassen, um z.B. bei einer ODHE anfallende Essigsäure aufzubereiten und als weiteres Wertprodukt in gewünschter Qualität zu gewinnen.

Grundsätzlich können die Produktströme oder daraus gebildete Ströme, d.h. Teilfolgeoder Folgeströme aus der ODHE und einem Olefinerzeugungsverfahren einem oder mehreren Reinigungs- und Zerlegungsteilen zugeführt werden. Diese Reinigungs- und Zerlegungsteile können jeweils unabhängig voneinander einen oder mehrere der o.g. Verfahrensschritte umfassen.

Ein erster bzw. zweiter Reinigungs- und Zerlegungsteil kann zunächst den ersten Produktstrom der ODHE oder den zweiten Produktstrom eines Olefinerzeugungsverfahrens verarbeiten, wobei jeweils ein entsprechender Folgestrom gebildet wird. Entsprechende Teilströme können als Teilprodukt- oder Teilfolgeströme gebildet und nur anteilig in folgende Verfahrensschritte überführt werden. Im Folgenden sollen die Begrifflichkeiten Produktstrom und Folgestrom sinngemäß auch solche Teilströme umfassen, wobei ein Teilprodukt- bzw. Teilfolgestrom einen Teil eines entsprechenden Produkt- bzw. Folgestroms darstellt.

Verfahren gemäß Ausgestaltungen der Erfindung umfassen mindestens einen (dritten) Reinigungs- und Zerlegungsteil. Dieser dritte Reinigungs- und Zerlegungsteil wird mit einem Strom gespeist, der durch Vereinigung der Folgeströme des ersten und zweiten Produktstroms bzw. entsprechender Teilfolgeströme entsteht.

Entsprechend der oben erläuterten Problemstellung stellt sich die vorliegende Erfindung die Aufgabe, wie bereits oben mit anderen Worten erwähnt, eine vorteilhafte Nutzung von leichten Kohlenwasserstoffen, insbesondere Ethan, aus den genannten Prozessen durch eine vorteilhafte Kombination von Olefinerzeugungsverfahren mit einer ODHE aufzuzeigen unter Berücksichtigung abweichender Kohlendioxid-, Sauerstoff- und Acetylengehalte.

Darüber hinaus können durch eine Integration entsprechender Anlagenteile, insbesondere in der Produkttrennung und -aufreinigung zusätzliche technische und wirtschaftliche Vorteile erreicht werden. Entsprechende Ausgestaltungen der Erfindung wurden zuvor mit A bis F bezeichnet.

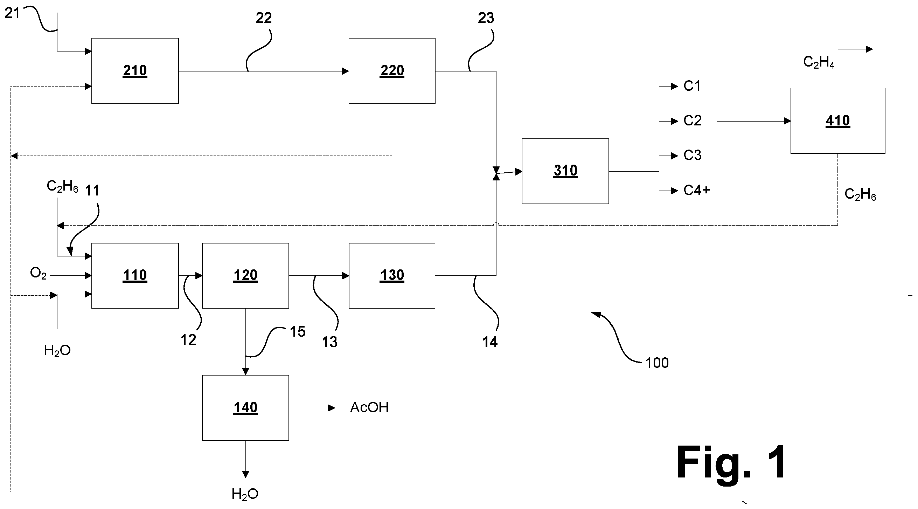

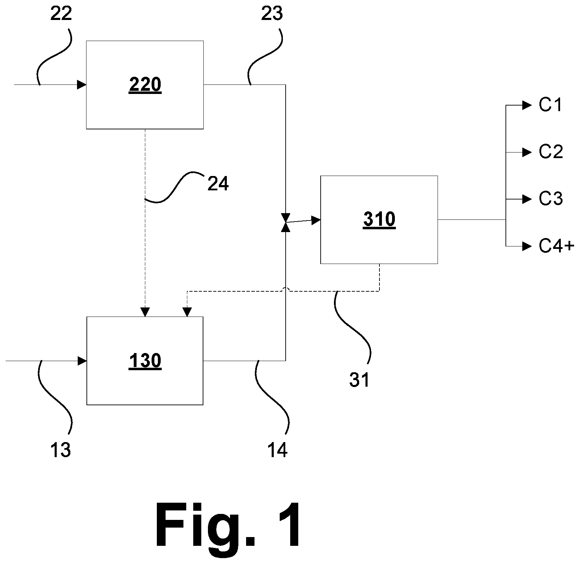

In Figur 1 ist ein Verfahren gemäß einer Ausgestaltung der vorliegenden Erfindung veranschaulicht und insgesamt mit 100 bezeichnet.

In dem Verfahren 100 werden einer katalytischen oxidativen Dehydrierung 110 die benötigten Reaktanden Ethan C₂H₆ und Sauerstoff O₂ zugeführt, wobei das Ethan hier auch als "erste Einsatzverbindung" bezeichnet wird. Zur Bereitstellung der entsprechenden Stoffströme können auch die weiter unten erläuterten Rückführströme eingesetzt werden. Zusätzlich kann bedarfsweise Wasserdampf H₂O als Verdünnungsmittel in die katalytische oxidative Dehydrierung 110 eingespeist werden. Das dafür benötigte Wasser kann zumindest anteilig aus der Raffinatwasserphase einer weiter unten erläuterten optionalen Essigsäureaufreinigung 140 oder aus einer entsprechenden wässrigen Fraktion aus einem zweiten Reinigungs- und Zerlegungsteil 220 stammen, wie mit gestrichelten Pfeilen veranschaulicht.

Der oxidativen Dehydrierung wird also Ethan C₂H₆, hier auch als "erste Einsatzverbindung" bezeichnet, in einem entsprechenden Einsatzstrom zugeführt, der hier auch als "erster Einsatzstrom" bezeichnet wird und mit 11 angegeben ist. Unter dem "ersten Einsatzstrom" kann dabei wahlweise ein (nur) Ethan enthaltender Stoffstrom C₂H₆, aber auch ein Mischstrom aus zumindest einem Teil der dargestellten Stoffströme C₂H₆, O₂ und H₂O usw. verstanden werden.

In der katalytischen oxidativen Dehydrierung 110 wird ein erster Produktstrom 12 gebildet, der Ethylen enthält, das hier als ein "erstes Produktolefin" bezeichnet wird, wobei der erste Produktstrom einer Kondensatabscheidung 120 unterworfen wird. In dieser werden eine im Wesentlichen Wasser und Essigsäure enthaltende Kondensatfraktion 15 und eine entsprechende, an Wasser und Essigsäure abgereicherte Fraktion gebildet, wobei letztere hier auch als ein "Folgestrom" des ersten Produktstroms 12 bezeichnet wird und mit 13 angegeben ist.

Die Kondensatfraktion 15 kann der erwähnten optionalen Essigsäureaufbereitung 140 unterworfen werden, in der eine im Wesentlichen Wasser enthaltende Wasserfraktion H₂O und eine im Wesentlichen Essigsäure enthaltende Essigsäurefraktion AcOH gebildet werden.

Stromab der Kondensatabscheidung 120 liegt der Folgestrom 13 in an Wasser und Essigsäure abgereicherter Form vor. Dieser kann nun bedarfsweise in einen ersten Reinigungs- und Zerlegungsteil 130 gespeist werden, der jeweils optional, und nicht gesondert dargestellt, eine Verdichtung, eine Rohgasbehandlung sowie eine Kohlendioxidentfernung umfassen kann.

Das stromab des ersten Reinigungs- und Zerlegungsteil 130 erhaltene Komponentengemisch, das hier ebenfalls als ein "Folgestrom" des ersten Produktstroms 12 und zur besseren Unterscheidbarkeit als "erster Folgestrom" bezeichnet wird und mit 14 angegeben ist, enthält zumindest einen Teil des Ethans, des Ethylens und der leichter als Ethylen siedenden Verbindungen aus dem ersten Produktgemisch. Es ist gegenüber dem ersten Produktstrom an Wasser und Essigsäure abgereichert. In der Ausführungsform B ist der erste Folgestrom an Sauerstoff und Acetylen abgereichert bzw. im Wesentlichen frei von diesen Komponenten. Der erste Folgestrom kann gegenüber dem ersten Produktstrom zumindest abgereichert an Kohlendioxid sein, wenn eine Kohlendioxidentfernung 133 zum Einsatz kommt (wie oben zu Ausführungsform C erläutert).

In dem Verfahren 100 wird ferner eine Sauerstoff und Kohlenstoff enthaltende zweite Einsatzverbindung in einem zweiten Einsatzstrom 21 unter Erhalt eines das erste Produktolefin, d.h. Ethylen, und/oder ein zweites Produktolefin, z.B. Propylen, enthaltenden zweiten Produktstroms 22 einem oder mehreren katalytischen Umsetzungsschritten 210 unterworfen. Der oder die katalytischen Umsetzungsschritte kann bzw. können beispielsweise im Zuge eines MTO-, MTP- oder FTTO-Verfahrens erläutert oder in einem Verfahren zur Hydrierung von Kohlendioxid, wie zuvor bereits mehrfach erläutert, durchgeführt werden. Unter dem "zweiten Einsatzstrom" kann dabei wahlweise ein (nur) die zweite Einsatzverbindung enthaltender Stoffstrom, aber auch ein Mischstrom aus mehreren Stoffströmen verstanden werden.

Der zweite Produktstrom umfasst insbesondere eine Mischung an Paraffinen und/oder Olefinen, insbesondere an Ethan und/oder Ethylen enthält. Bedarfsweise kann auch dem Olefinerzeugungsverfahren 210 Wasser oder Wasserdampf zugeführt, dies ist aber in der Regel nicht der Fall. Der zweite Produktstrom 22 kann optional einen zweiten Reinigungs- und Zerlegungsteil 220 durchlaufen, wobei sich ein "Folgestrom" des zweiten Produktstroms 22 ergibt, der hier auch als "zweiter Folgestrom" bezeichnet wird. Falls dem Olefinerzeugungsverfahren 210 Wasser oder Wasserdampf H₂O, z.B. als Verdünnungsmittel zugesetzt werden sollen, kann dies in gleicher Weise wie oben für die katalytische oxidative Dehydrierung 110 beschrieben erfolgen.

Die Folgeströme 14 und 23 werden sodann vereinigt und einem gemeinsamen dritten Reinigungs- und Zerlegungsteil 310 zugeführt. Dieser kann insbesondere die bereits aufgeführten Verfahrensschritte, insbesondere aber geeignete Fraktionierungen enthalten. Hier wird insbesondere eine nicht gesondert veranschaulichte Kohlendioxidentfernung 313 durchlaufen, die insbesondere eine Laugewäsche (bei den erläuterten Ausführungsformen A, B und C), aber optional auch eine regenerative Wäsche enthalten kann (Ausführungsform D). Insbesondere enthält jedoch nur der erste Reinigungs- und Zerlegungsteil 130 oder der dritte Reinigungs- und Zerlegungsteil 130 eine solche regenerative Wäsche und/oder Laugewäsche, so dass eine Doppelung von Verfahrensschritten vermieden wird. Ausgestaltungen können aber auch umfassen, dass eine Kohlendioxidentfernung in dem ersten Reinigungs- und Zerlegungsteil 130 als Wäsche durchgeführt wird, in der eine Ablauge aus einer nachgeschalteten Kohlendioxidentfernung in dem dritten Reinigungs- und Zerlegungsteil 310 eingesetzt werden kann. Grundsätzlich kann in diesen und anderen Ausführungsformen auch eine zusätzliche weitere optionale Laugewäsche als Feinreinigung enthalten sein.

In dem dritten Reinigungs- und Zerlegungsteil 310 werden, gleichzeitig oder nacheinander, und in an sich bekannter Reihenfolge, eine Methanfraktion C1, die auch leichter als Methan siedende Verbindungen enthalten kann, eine Fraktion C2 mit Kohlenwasserstoffen mit zwei Kohlenstoffatomen, eine Fraktion C3 mit Kohlenwasserstoffen mit drei Kohlenstoffatomen, und eine Fraktion C4 mit Kohlenwasserstoffen mit vier und ggf. mehr Kohlenstoffatomen gebildet.

Die im dritten Reinigungs- und Zerlegungsteil 310 erhaltene Fraktion C2 mit Kohlenwasserstoffen mit zwei Kohlenstoffatomen kann einer Trennung dieser Kohlenwasserstoffe voneinander (Split) 410 unterworfen werden. Das hier erhaltene Ethan C₂H₆ kann ganz oder anteilig als Reaktionseinsatz der katalytischen oxidativen Dehydrierung 110 verwendet werden und Ethylen C2H4 wird spezifikationsgerecht (insbesondere in Bezug auf Kohlendioxid-, Sauerstoff- und Acetylengehalt) als Wertprodukt gewonnen. Das für die ODHE benötigte Ethan C₂H₆ kann dabei also anteilig oder ausschließlich aus dem Rückführstrom der Trennung 410 bereitgestellt werden.

In Figur 2 sind Aspekte eines Verfahrens gemäß einer Ausgestaltung der vorliegenden Erfindung veranschaulicht, wobei die stromauf des ersten und zweiten Reinigungs- und Zerlegungsteils 130, 220 und stromab des dritten Reinigungs- und Zerlegungsteils 310 vorgesehenen Verfahrensschritte nicht dargestellt sind. Die Einbindung ergibt sich aus den auch hier dargestellten Stoffströmen 13, 22, C1, C2, C3 und C4+

Figur 2 veranschaulicht dabei die in den erläuterten Ausführungsformen E bzw. F in den ersten Reinigungs- und Zerlegungsteil 130 geführten und dort vorteilhaft genutzten Ströme. Es handelt sich einerseits in der Ausführungsform E um ein Kondensat 24 aus dem katalytischen Olefinerzeugungsverfahren 210, das im zweiten Reinigungs- und Zerlegungsteil 220 aus dem zweiten Produktstrom 22 abgetrennt wird, sowie andererseits in der Ausführungsform F um eine Ablauge 31 aus einer Laugewäsche im dritten Reinigungs- und Zerlegungsteil 310.

## Patentansprüche

1. Verfahren (100) zur Herstellung eines oder mehrerer Syntheseprodukte, bei dem
- Ethan als eine erste Einsatzverbindung in einem ersten Einsatzstrom (11) unter Erhalt eines Ethylen als ein erstes Produktolefin enthaltenden ersten Produktstroms (12) einer oxidativen Dehydrierung (110) unterworfen wird,
- eine Sauerstoff und Kohlenstoff enthaltende zweite Einsatzverbindung in einem zweiten Einsatzstrom (21) unter Erhalt eines das erste und/oder ein zweites Produktolefin enthaltenden zweiten Produktstroms (22) einem oder mehreren katalytischen Umsetzungsschritten (210) unterworfen wird, **dadurch gekennzeichnet, dass**
- zumindest ein Teil des ersten Produktstroms (12) unter Erhalt eines ersten Folgestroms (13, 14) einem oder mehreren ersten Reinigungs- und/oder Trennschritten (130) unterworfen wird,
- zumindest ein Teil des zweiten Produktstroms (21) unter Erhalt eines zweiten Folgestroms (22, 23) keinem, einem oder mehreren zweiten Reinigungsund/oder Trennschritten (310) unterworfen wird,
- zumindest ein Teil des ersten Folgestroms (13, 14) und zumindest ein Teil des zweiten Folgestroms (22, 23) zur Bildung eines dritten Einsatzstroms verwendet wird, wobei der dritte Einsatzstrom einem oder mehreren dritten Reinigungs- und/oder Trennschritten (310) zugeführt wird.

2. Verfahren (100) nach Anspruch 1, bei dem der oder die dritten Reinigungsund/oder Trennschritte (310) eine Kohlendioxidentfernung umfassen, wobei die Bildung des ersten Folgestroms (13, 14) und des zweiten Folgestroms (23, 24) ohne eine Durchführung einer Kohlendioxidentfernung erfolgt.

3. Verfahren (100) nach Anspruch 2, bei dem die von dem oder den dritten Reinigungs- und/oder Trennschritten (310) umfasste Kohlendioxidentfernung in Form einer Laugewäsche durchgeführt wird.

4. Verfahren (100) nach Anspruch 2 oder 3, bei dem die Bildung des ersten Teil- oder Folgestroms (13, 14) ohne eine Rohgasbehandlung und/oder Kohlendioxidentfernung durchgeführt wird.

5. Verfahren (100) nach Anspruch 2, bei dem die Bildung des ersten Teil- oder Folgestroms (13, 14) eine Rohgasbehandlung umfasst.

6. Verfahren (100) nach einem der Ansprüche 2 bis 5, bei dem die Bildung des ersten Teil- oder Folgestroms (13, 14) eine Druckerhöhung umfasst.

7. Verfahren (100) nach einem der Ansprüche 3 bis 5, bei dem die Bildung des ersten Teil- oder Folgestroms (13, 14) eine Entfernung von Kohlendioxid umfasst.

8. Verfahren (100) nach einem der Ansprüche 2 bis 7, bei dem die von dem oder den dritten Reinigungs- und/oder Trennschritten (310) umfasste Kohlendioxidentfernung eine Aminwäsche umfasst.

9. Verfahren (100) nach einem der Ansprüche 2 bis 8, bei dem in dem einen oder den mehreren katalytischen Umsetzungsschritten (210) anfallendes Prozesswasser in einem oder mehreren der ersten Reinigungs- und/oder Trennschritte (130) verwendet wird.

10. Verfahren (100) nach einem der Ansprüche 2 bis 9, bei dem eine Ablauge aus der von dem oder den dritten Reinigungs- und/oder Trennschritten (310) umfassten Kohlendioxidentfernung in einem oder mehreren der ersten Reinigungs- und/oder Trennschritte (130) verwendet wird.

11. Anlage zur Herstellung eines oder mehrerer Syntheseprodukte, die dafür eingerichtet ist,
- Ethan als eine erste Einsatzverbindung in einem ersten Einsatzstrom (11) unter Erhalt eines Ethylen als ein erstes Produktolefin enthaltenden ersten Produktstroms (12) einer oxidativen Dehydrierung (110) zu unterwerfen,
- eine Sauerstoff und Kohlenstoff enthaltende zweite Einsatzverbindung in einem zweiten Einsatzstrom (21) unter Erhalt eines das erste und/oder ein zweites Produktolefin enthaltenden zweiten Produktstroms (22) einem oder mehreren katalytischen Umsetzungsschritten (210) zu unterwerfen, **gekennzeichnet durch** Mittel, die dafür eingerichtet sind,
- zumindest einen Teil des ersten Produktstroms (12) unter Erhalt eines ersten Folgestroms (13, 14) einem oder mehreren ersten Reinigungs- und/oder Trennschritten (310) zu unterwerfen,
- zumindest einen Teil des zweiten Produktstroms (21) unter Erhalt eines zweiten Folgestroms (22, 23) keinem, einem oder mehreren zweiten Reinigungs- und/oder Trennschritten (310) zu unterwerfen,
- zumindest einen Teil des ersten Folgestroms (13, 14) und zumindest einen Teil des zweiten Folgestroms zur Bildung eines dritten Einsatzstroms zu verwenden und den dritte Einsatzstrom einem oder mehreren dritten Reinigungs- und/oder Trennschritten (310) zuzuführen.

12. Anlage nach Anspruch 11, die zur Durchführung eines Verfahrens nach einem der Ansprüche 1 bis 10 eingerichtet ist.
